# EUROPÄISCHE PATENTSCHRIFT

(11) **EP 2 854 920 B1**
(45) Veröffentlichungstag und Bekanntmachung des Hinweises auf die Patenterteilung: **06.04.2016**
(21) Anmeldenummer: 13728131.7
(22) Anmeldetag: 29.05.2013
(51) Int. Cl.: A61M 16/04, A61M 16/08, B29C 45/36

(54) **LARYNXMASKE MIT EINEM SUPRAGLOTTISCHEN TUBUS**
LARYNGEAL MASK WITH SUPRAGLOTTIC TUBE
MASQUE LARYNGÉAL AVEC TUBE SUPRAGLOTTIQUE

(30) Priorität: 04.06.2012 CH 7672012
(43) Veröffentlichungstag der Anmeldung: 08.04.2015
(73) Patentinhaber: Singularity AG, 8124 Maur (CH)
(72) Erfinder: DUBACH, Werner F., 8124 Maur (CH)
(74) Vertreter: Schneider Feldmann AG Patent- und Markenanwälte
(86) Internationale Anmeldenummer: PCT/EP2013/061017
(87) Internationale Veröffentlichungsnummer: WO 2013/182457

(56) Entgegenhaltungen:
- WO-A1-2012/042219
- WO-A2-2004/016308
- US-A- 4 584 998
- US-A1- 2005 051 175
- US-A1- 2006 032 505
- US-A1- 2012 090 609

## Beschreibung

Die vorliegende Erfindung betrifft eine Larynxmaske mit einem supraglottischen Tubus zum Einführen eines Larynxmaskenkopfes über den Kehlkopf, wobei der supraglottische Tubus drei Lumen aufweist, nämlich einen Respirationslumen der der Beatmungsluftzufuhr und der Instrumentierung dient, einen oesophagealen Lumen, welcher dem oesophagealen Zugang dient und ein medial dazwischen verlaufender proximal geschlossener Führungslumen.

Larynxmaske bestehen aus Kunststoff und werden meist nur einmal gebraucht. Die Larynxmaske besteht üblicherweise aus einem supraglottischen Tubus und einem Larynxmaskenkopf. Diese beiden Teile werden getrennt gefertigt und danach schweisstechnisch oder durch Kleben miteinander verbunden. Ein typisches Beispiel einer solchen Larynxmaske zeigt die US 2003/037790. Hier weist der supraglottische Tubus zwei parallele Lumen auf, wobei das eine Lumen das Respirationslumen und das weite Lumen das Oesophageallumen ist. Dem gegenüber zeigt die US 2006/032505 einen supraglottischen Tubus auf, der durch drei nebeneinander verlaufenden Schläuche gebildet ist. Aus der US 2007/028923 ist eine Larynxmaske bekannt, deren supraglottischer Tubus einen zentralen Respirationslumen hat und in der Wandung einmal dorsal und einmal ventral je einen Tubus mit geringem Durchmesser gezeigt ist. Obwohl weder aus der Beschreibung noch aus den Zeichnungen hervorgeht, dass der Tubus und der Larynxmaskenkopf getrennt gefertigt worden sind, lässt allein die Konstruktion schon keinen anderen Schluss zu. Der gekrümmte Verlauf des Tubus verunmöglicht nämlich das Herausziehen eines die Lumen bildenden Kerns. Ebenso lässt der Tubus, der in der Wand eingebettete Lumen aufweist, sich nicht anders fertigen als durch Strangpressen oder Extrasion. Ein solcher dickwandiger Schlauch lässt sich später durch Wärmebehandlung nach Wunsch auch noch formen.

Die Erfindung geht von einer Larynxmaske gemäss der WO 2010/060227 aus. Diese Larynxmaske besitzt einen supraglottischen Tubus an dem der Larynxmaskenkopf befestigt ist. Der Larynxmaskenkopf hat entsprechend eine Aufnahmebuchse, in den der Tubus eingeschoben und mit dem Larynxmaskenkopf verschweiss- oder verklebbar ist. Eine solche Fertigung verlangt viel Handarbeit, ist entsprechend zeitaufwändig und kostenintensiv. Der hier zum Einsatz kommende supraglottische Tubus wird hier spritzgusstechnisch gefertigt. Ebenso wird der Larynxmaskenkopf mit einer zweiten Spritzgussform ebenso zweiteilig gefertigt. Die spritzgusstechnische Fertigung des supraglottischen Tubus, der üblicherweise eine Länge von ca. 20 cm aufweist, lässt sich spritzgusstechnisch deshalb herstellen, weil einerseits die Wandstärken keine relevanten Dickenunterschiede aufweisen und zum anderen weil die, die Lumen bildende Kerne als Züge oder Schieber in einem Teil des Werkzeuges beweglich gehalten sind, während im anderen Werkzeugteil die Enden der Kerne absolut kraft- und formschlüssig gehalten werden können und somit keine Verformung der Kerne auftreten kann. Es scheint daher naheliegend, um sowohl den supraglottischen Tubus als auch den Larynxmaskenkopf zusammen einstückig zu fertigen. Dies hat sich aber bisher als scheinbar nicht realisierbar gezeigt, da sowohl im supraglottischen Tubus als auch im Larynxmaskenkopf entsprechende bewegliche Kerne erforderlich sind und nun diese Kerne im Gegenwerkzeugteil nicht mehr form- und kraftschlüssig abgefangen werden können. Bei dem herrschenden Druck in solchen Kunststoff spritzgussformen lassen sich die Kerne nicht einfach flächig an ihren Enden aufeinanderdrücken. Würde man, bei einer solchen Konstruktion die Form mit Kunststoff füllen, würden bei den herrschenden beziehungsweise auftretenden Drücken sich die Kerne spätestens nach wenigen tausend Öffnungs- und Schliessvorgänge der Spritzgussform sich nicht mehr einwandfrei schliessen und an den Stossstellen der Kerne würden sich Filme oder wie dies in der Fachsprache heisst Schwimmhäuten bilden. Mit anderen Worten, man müsste die fertigen Larynxmasken prüfen, um festzustellen, dass sowohl das Respirationslumen als auch das Oesophageallumen einen ungehinderten Durchgang aufweist. Dies würde aber wiederum zusätzliche Arbeitgänge, zusätzliche Prüfvorrichtungen und somit vermehrte Kosten verursachen. Es ist daher die Aufgabe der vorliegenden Erfindung eine Larynxmaske aus Kunststoff einstückig zu fertigen unter Meidung der beschriebenen Probleme.

Diese Aufgabe löst eine Larynxmaske der eingangs genannten Art, welche sich dadurch auszeichnet, dass der supraglottische Tubus und die Larynxmaske einstückig spritzgusstechnisch gefertigt sind, wobei zwischen einem lateralen Lumen und dem medialen Führungslumen eine über mindestens einen Teil der Länge dem supraglottischen Tubus vom proximalen Ende her ein die beiden Lumen verbindender Schlitz verläuft.

Dieser erwähnte erfindungsgemässe Schlitz lässt sich nur bilden wenn das Spritzgussformwerkzeug, welches hierzu erforderlich ist, einen Steg aufweist zwischen jenem Kern zur Bildung des Führungslumens und einer der benachbarten Kerne, wobei vom proximalen Ende an in distaler Richtung diese beiden Kerne über einen Stabilisierungssteg miteinander verbunden sind.

Dank dieser Lösung ist insbesondere der Kern zur Bildung des Führungslumens zusätzlich stabilisiert, was besonders wesentlich ist, da dieser Kern ein sogenannter fliegender Kern ist, der sich weder am gegenüberliegenden Werkzeugteil noch an einen Gegenkern abstützt. Nur so lässt sich der distal geschlossene Führungslumen bilden.

Da die bisher bekannten Larynxmasken mit Ausnahme der Lösung gemäss der WO 2010/060227 keinen medialen Führungslumen aufweisen, lassen sich bei den sonst bekannten Larynxmasken eine solche Lösung nicht realisieren. Besteht beispielsweise der supraglottische Tubus lediglich aus einem Respirationslumen und einem parallel dazu verlaufenden oesophagealen Lumen, so darf zwischen diesen beiden Lumen keine Verbindung bestehen. Also insbesondere auch kein Schlitz, der diese beiden Lumen verbindet, vorhanden sein, damit über den Oesophageallumen abzusaugendes Material nicht in den Respirationslumen gelangen darf. Nur bei einer Larynxmaske mit einem supraglottischen Tubus wie eingangs beschreiben, lässt sich das erfindungsgemässe Problem lösen.

In der Zeichnung sind unterschiedliche Ausführungsformen dargestellt, wobei die Unterscheidung im Wesentlichen nur in der Ausgestaltung des Larynxmaskenkopfes besteht. Hierbei soll klar gemacht werden, dass die erfindungsgemässe Lösung für verschiedene Arten von Larynxmasken geeignet sind, solange diese einen supraglottischen Tubus der eingangs genannten Art aufweisen.

In der Zeichnung sind bevorzugte Ausführungsbeispiele des Erfindungsgegenstandes dargestellt und anhand der nachfolgenden Beschreibung mit Bezug auf die Zeichnung erläutert. Es zeigt:
- Figur 1: eine einteilige Larynxmaske mit Blick auf die ventrale Seite in Ansicht.
- Figur 2: zeigt einen zentrischen Vertikalschnitt entlang der Line D-D in Figur 1 und
- Figur 3: einen ebensolchen Schnitt entlang der Linie E-E mit Blick auf die Trennwand zwischen Oesophageallumen und Führungslumen, wobei es sich bei den Figuren 1 bis 3 um Darstellungen einer Larynxmaske mit aufblasbaren Cuff handelt. In
- Figur 4: ist eine Larynxmaske in einem horizontalen Schnitt gezeigt, wobei es sich hier um eine Larynxmaske handelt, deren Cuff hohlraumfrei also aus vollem Material gefertigt ist.
- Figur 5: zeigt dieselbe Larynxmaske nach Figur 4 in der Aufsicht mit Blick von der dorsalen Seite auf die Larynxmaske.
- Figur 6: zeigt die Larynxmaske bei einem Schnitt entlang der Linie E-E in Figur 5, wobei der supraglottische Tubus senkrecht zu dessen Verlaufsrichtung geschnitten ist, während der Larynxmaskenkopf in Ansicht von dieser Schnittfläche aus gesehen dargestellt ist.
- Figur 7: zeigt den Larynxmaskenkopf in einem Vertikalschnitt entlang der Linie D-D in der Figur 5.

Die Figuren 8 bis 10 zeigen eine Variante der Larynxmaske gemäss den Figuren 4 bis 7, wobei hier der Larynxmaskenkopf in medial-lateral Richtung federnd gestaltet ist.

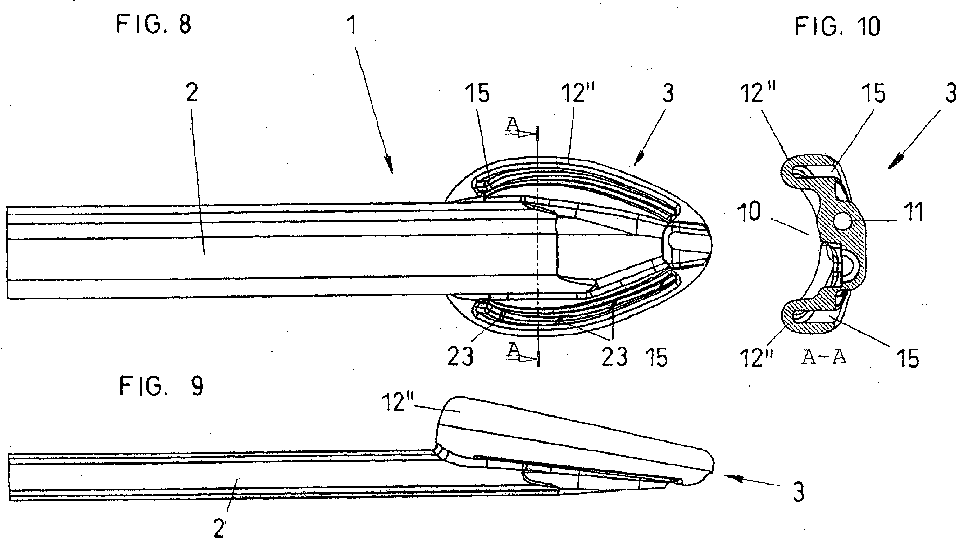

- Figur 8: zeigt diese Larynxmaske in einer dorsalen Ansicht und
- Figur 9: in einer lateralen Ansicht, während
- Figur 10: einen Vertikalschnitt durch den Larynxmaskenkopf entlang der Linie A-A in Figur 8 zeigt.

Die nachfolgend der Lagebestimmung dienenden Begriffe beziehen sich patientenbezogen auf die Lage der Larynxmaske im Patienten. Entsprechend bedeutet distal von der Körpermitte des Patienten weg gelegen, während proximal zur Körpermitte des Körpers hin gerichtet. Entsprechend bedeutend hier lateral von der Körpermitte abgewandt, während medial zur Körpermitte hin bedeutet.

In dieser Patentanmeldung wird der Begriff Larynxmaske für die Gesamtheit bestehend aus dem supraglottischen Tubus und dem damit verbundenen Larynxmaskenkopf verstanden. Die Larynxmaske, also die Gesamtheit besteht folglich aus dem supraglottischen Tubus 2 und den damit einstückig verbundenen Larynxmaskenkopf 3.

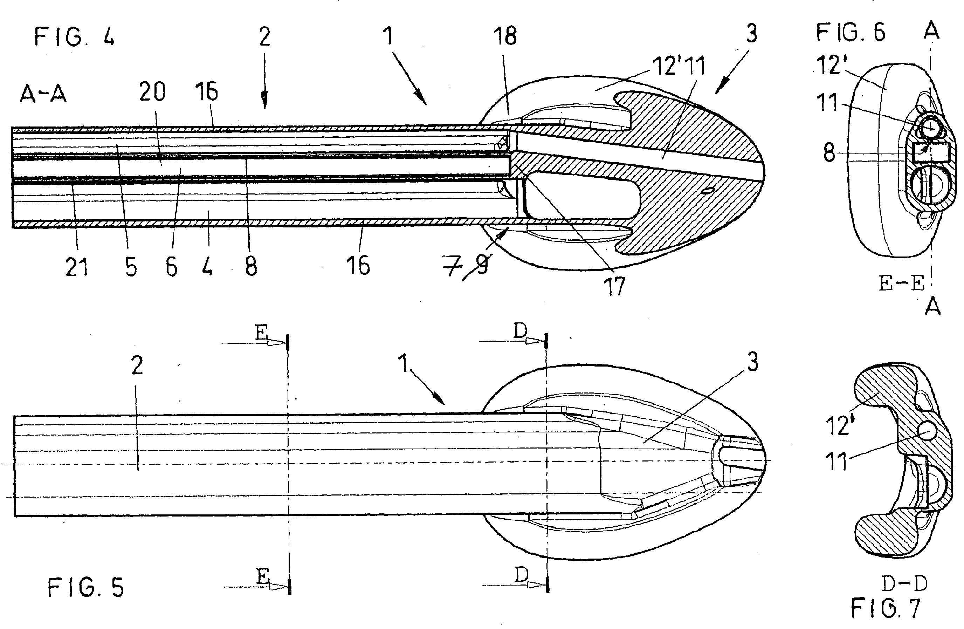

Der supraglottische Tubus ist folglich der Tubus, der über beziehungsweise oberhalb der Glottis hinwegführbar ist. Der supraglottische Tubus 2 besitzt mehrere Lumen. In der erfindungsgemässen Ausführung hat der supraglottische Tubus 2 drei Lumen. Hierzu wird auf die Figur 4 verwiesen. Das Lumen mit dem grössten Durchmesser beziehungsweise mit der grössten lichten Weite ist der lateral liegende Respirationslumen 4. Dieser grenzt an den medial verlaufenden Führungslumen 6. Wiederum lateral auf der anderen Seite des Führungslumens 6 verläuft das somit laterale Oesophageallumen 5. Der Querschnitt des supraglottischen Tubus 2 ist in der Figur 6 im Schnitt ersichtlich, wobei dieser Schnitt entlang der Linie E-E in der Figur 5 verläuft. Diese Figur 5 zeigt die Larynxmaske 1 mit Blick auf die ventrale Seite der Larynxmaske. Die Figur 4 ist nun ein Horizontalschnitt, der in der Mitte der Höhe des supraglottischen Tubus 2 verläuft. Diese Schnittebene A-A ist in der Figur 6 dargestellt.

Der supraglottische Tubus 2 geht trennstellenfrei in den Larynxmaskenkopf 3 über. Der Bereich in dem der supraglottische Tubus 2 in den Larynxmaskenkopf 3 übergeht, wird als Übergangsbereich 18 bezeichnet. In diesem Übergangsbereich 18 ist die proximale Endwand 17 vorhanden, welche das Führungslumen 6 proximal abschliesst. Im gleichen Übergansbereich 18 geht das Oesophageallumen 5 im Bereiche des Larynxmaskenkopfes 3 in den Oesophagealdurchgang 11 über. Dieser Oesophagealdurchgang 11 verläuft oberhalb des Respirationsraumes 10, wie dies am deutlichsten in der Figur 7 ersichtlich ist, welche einen Schnitt durch den Larynxmaskenkopf 3 im Bereich der Schnittlinie D-D in Figur 5 zeigt. In dieser Figur ist der Oesophagealdurchgang 11 als zylindrische Bohrung erkennbar, der wie erwähnt oberhalb des Respirationsraumes 10 verläuft. Deutlich erkennbar ist in der Figur 4 auch, dass sich der Oesophagealdurchgang 11 absolut geradlinig durch den Larynxmaskenkopf 3 erstreckt und im erwähnten Übergangsbereich 18 dieser Oesophagealdurchgang 11 in das Oesophageallumen 5 endet. Im Übergangsbereich 18 ergibt sich hier eine Richtungsänderungsstelle 19. Aus dieser Darstellung ist auch klar ersichtlich, dass die Länge des Oesophagealdurchganges 11 rund ein Drittel oder mehr der Länge des supraglottischen Tubuses 2 entspricht.

Im selben Übergangsbereich 18 mündet auch das Respirationslumen 4 in den Respirationsraum 10, wie dies sowohl in der Figur 4 als auch in der Figur 7 ersichtlich ist. Bei dieser Ausführungsform gemäss den Figuren 4 bis 6 handelt es sich um eine Lösung bei der der Cuff 12 als hohlraumfreier Cuff 12' gestaltet ist. Ein hohlraumfreier Cuff 12' ergibt eine etwas erhöhte Festigkeit, was die Einführung der Larynxmaske in den Patienten erleichtert. Larynxmasken dieser Bauart sind insbesondere für die Verwendung im Notfallbereich sinnvoll.

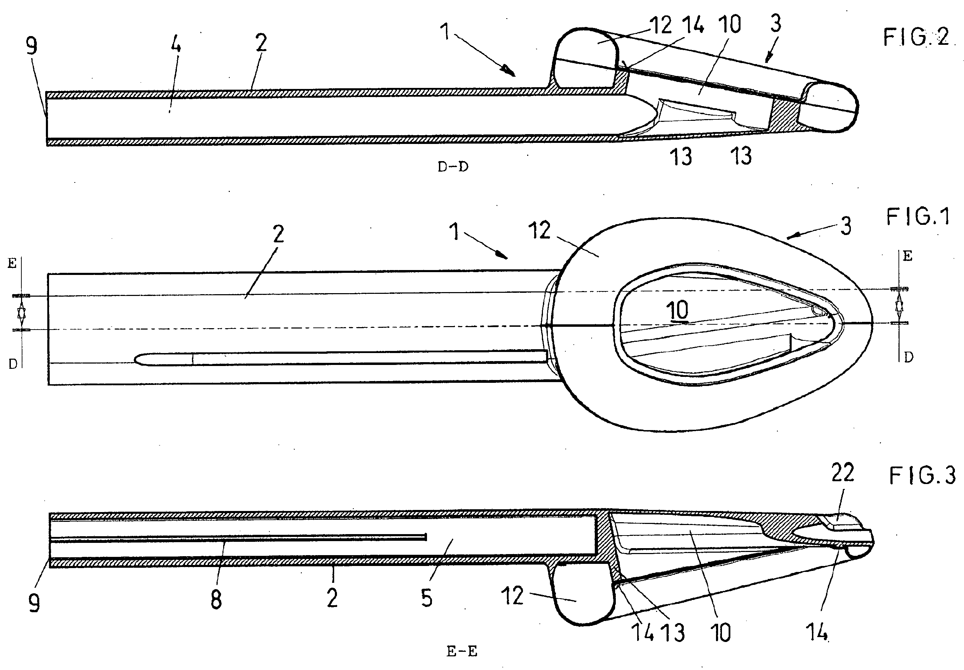

In den Figuren 1 bis 3 ist eine Larynxmaske in einem aufblasbarem Cuff 12' dargestellt. Die hier gezeigte Lösung, die heute insbesondere im klinischen Bereich eingesetzt wird, ist wiederum einstückig gefertigt. Auch hier ist wiederum die Larynxmaske insgesamt mit 1 bezeichnet, während der supraglottische Tubus mit 2 und der Larynxmaskenkopf mit 3 bezeichnet ist. Die Larynxmaske 1 ist hier im Zustand gezeigt, in der die Larynxmaske 1 aus der Spritzgussform kommt. Der Cuff 12 ist entsprechend noch offen und muss durch eine Schweissung oder Klebung geschlossen werden. Der Cuff 12 umgibt den Respirationsraum 10 und endet in einen nach innen gerichteten, das heisst zum Zentrum des Respirationsraumes 10 hin gerichteten Schweissrand 14. Der Respirationsraum 10 selbst, wird von einer umlaufenden Klebe- bzw. Schweisswand 13 begrenzt. Der bereits erwähnte, kragenartige Klebe- bzw. Schweissrand 14, ist im fertig montierten Zustand auf der Klebe- bzw. Schweisswand 13 dichtend aufgeklebt oder aufgeschweisst. Deutlicher als in den Versionen gemäss den Figuren 4 bis 7, ist hier insbesondere in der Figur 3 der erfindungsgemässe Schlitz 8 erkennbar. Dieser Schlitz 8 verläuft vom distalen Ende 9 des supraglottischen Tubus 2 in proximaler Richtung zum Larynxmaskenkopf 3 hin. Die Länge dieses Schlitzes 8 beträgt mindestens 1/3 der gesamten Länge des supraglottischen Tubus 2. Der Schlitz 8 kann in einem der beiden Trennwände 20, 21 entweder in der Trennwand 20, welche das Führungslumen 6 vom Oesophageallumen 5 trennt, oder in der Trennwand 21, welche das Führungslumen 6 vom Respirationslumen 4 trennt, angeordnet sein. Sowohl in der Ausführung gemäss den Figuren 1 bis 3 als auch in der Version gemäss den Figuren 4 bis 7 verläuft der Schlitz in der Trennwand 20, welche das Führungslumen 6 vom Oesophageallumen 5 trennt. Der Oesophageallumendurchgang mündet in beiden Versionen vor dem Cuff 12 am proximalen Ende der Larynxmaske und verläuft über den Cuff hinweg in einer offenen Rinne 22. Der Schlitz 8 wird von selbst gebildet, dadurch, dass zwischen ihrem Kern, der einer der beiden lateralen Lumen 4 oder 5 bildet und dem medialen Führungslumen 6 ein Verbindungssteg in der Spritzgusswerkzeugform vorhanden ist. Dank diesem Steg, sind die beiden im Durchmesser kleinsten Kerne gegeneinander abgestützt und entsprechend versteift. Da aber insbesondere der mediale Kern des Spritzgussformwerkzeuges problematisch ist, da dieser nicht an einem Gegenkern sich abstützen kann, kann der Schlitz 8 auch in der Trennwand 21 zwischen dem Führungslumen 6 und dem Respirationslumen 4 verlaufen. Jener Kern des Spritzgussformwekzeuges, mit dem die erfindungsgemässe Larynxmaske gefertigt wird, ist so gestaltet, dass ein weiterer Kern, mit dem der Oesophagealdurchgang 11 gebildet wird, mit jenem Kern, der das Oesophageallumen 5 bildet, im Stossbereich formschlüssig ineinander greifen können. Entsprechend wird der eine Kern an seinem Ende, welches auf den anderen Kern zuweist, eine Bohrung und der andere Kern einen darin formschlüssig passenden Zapfen aufweisen, sodass sich diese beiden Kerne versteifend gegenseitig fixieren.

In einer letzten Ausführungsform der Erfindung, wobei hier es sich auch hier um eine einstückig gefertigte Larynxmaske handelt, und bei dem der supraglottischen Tubus 2 genau gleich gestaltet ist wie bei den zuvor beschriebenen beiden Ausführungen, ist ein Larynxmaskenkopf 3 mit einer anders gestalteten Cuff 12'' dargestellt. Diese Lösung kombiniert die Vorteile der beiden zuvor beschriebenen Lösungen mit dem Cuff 12 bzw. mit der Lösung gemäss dem Cuff 12'. Hier handelt es sich nicht um einen aufblasbaren Cuff, sondern um im Prinzip um einen Cuff ohne Hohlraum, der aber trotzdem in Lateral- Medialrichtung federnd gestaltet ist. Auch hier sind gleiche Teile mit gleichen Bezugszahlen versehen, wie bei der zuvor beschriebenen Variante. Der Cuff 12" hat hier parallel zu seiner peripheren Umrisslinie im lateralen Bereich eine im Querschnitt U-förmige Federrinne 15. Diese Federrinne erlaubt die äussere laterale Wandbereiche von lateral zu medial zu federn. Dies erlaubt eine Breitenanpassung des Larynxmaskenkopfes, ohne dass dieser hierzu aufgeblasen werden muss. Die Federkraft der Anpassung in lateralmedialer Richtung lässt sich mit zusätzlichen Gestaltungsmitteln anpassen. Hierzu können in der im Querschnitt U-förmigen Federrinne 15 mehrere dünnwandige quer zur Verlaufsrichtung der Federrinne 15 verlaufende Federwände 23 eingeformt sein (siehe Figur 8). Solche Federwände 23 können senkrecht zur Verlaufsrichtung der Federrinne 18 angeordnet sein, oder auch zu deren Längsrichtung schräg verlaufend, wie hier in der Zeichnung dargestellt, angeordnet sein. Ebenso brauchen solche Federwände nicht geradlinig sondern können gekrümmt, bogenförmig oder auch S-förmig verlaufend gestaltet sein. All diese Varianten ergeben Möglichkeiten die Federkraft passend zu gestalten.

Die verschiedenen Ausführungsformen sollen lediglich belegen, dass das Konzept der einstückigen Fertigung der Larynxmaske viele Varianten bezüglich der Ausgestaltung des Larynxmaskenkopfes zulässt. Wesensgrundlage jedoch um eine solche einstückige Fertigung überhaupt zu ermöglichen, beruht darauf, dass einer der beiden Trennwände zwischen einem der beiden lateralen Lumen, nämlich dem Respirationslumen 4 oder dem Oesophageallumen 5 und dem medialen Führungslumen 6 über einen Schlitz 8 miteinander verbunden sind.

### Bezugszeichenliste:

- 1: Larynxmaske
- 2: Tubus
- 3: Larynxmaskenkopf
- 4: Respirationslumen
- 5: oesophagealer Lumen
- 6: Führungslumen
- 7: proximales Ende
- 8: Schlitz
- 9: distales Ende
- 10: Respirationsraum
- 11: Oesophagealdurchgang
- 12: Cuff
- 12': Cuff
- 12": Cuff
- 13: Schweisswand
- 14: Schweissrand
- 15: Federrinne
- 16: laterale Wand
- 17 18: Übergangsbereich
- 19: Richtungsänderungsstelle

## Patentansprüche

1. Larynxmaske (1) mit einem supraglottischen Tubus (2) zum Einführen eines Larynxmaskenkopfes (3) über den Kehlkopf, wobei der supraglottische Tubus drei Lumen aufweist, nämlich einen Respirationslumen (4) der der Beatmungsluftzufuhr und der Instrumentierung dient, einen oesophagealen Lumen (5), welcher dem oesophagealen Zugang dient und ein medial dazwischen verlaufender proximal geschlossener Führungslumen (6), **dadurch gekennzeichnet, dass** der supraglottische Tubus (2) und der Larynxmaskenkopf (3) einstückig spritzgusstechnisch gefertigt sind, wobei zwischen einem lateralen Lumen (4,5) und dem medialen Führungslumen (6) eine über mindestens einen Teil der Länge der supraglottischen Tubus (2) vom distalen Ende (9) her ein die beiden Lumen verbindender Schlitz (8) verläuft.

2. Larynxmaske (1) nach Anspruch 1, **dadurch gekennzeichnet, dass** der verbindende Schlitz (8) das Respirationslumen (4) mit dem Führungslumen (6) verbindet.

3. Larynxmaske (1) nach Anspruch 1, **dadurch gekennzeichnet, dass** der verbindende Schlitz (8) das oesophageale Lumen (5) mit dem Führungslumen (6) verbindet.

4. Larynxmaske (1) nach Anspruch 1, **dadurch gekennzeichnet, dass** die Länge des Schlitzes (8) sich mindestens über ein Viertel, bevorzugterweise mindestens ein Drittel der gesamten Länge des Tubus (2) vom proximalen (7) zum distalen Ende (9) hin erstreckt.

5. Larynxmaske (1) nach Anspruch 1, **dadurch gekennzeichnet, dass** die einteilige Larynxmaske (1) im Larynxmaskenkopf (3) einen zentralen Respirationsraum (10) aufweist, in den das Respirationslumen (4) des Tubus (2) mündet und oberhalb des Respirationsraumes (10) ein Oesophagealdurchgang (11) verläuft, in den der Oesophageallumen (5) mündet, und dass der Respirationsraum(10) von einem Cuff (12) umgeben ist.

6. Larynxmaske (1) nach Anspruch 5, **dadurch gekennzeichnet, dass** der Cuff (12) ein aufblasbarer Cuff ist.

7. Larynxmaske (1) nach Anspruch 5, **dadurch gekennzeichnet, dass** der Respirationsraum (10) von einer umlaufenden Klebe- bzw. Schweisswand (13) begrenzt ist, und dass der offen gespritzte Cuff (12) einen umlaufenden der Form und Grösse der Klebe- bzw. Schweisswand (13) angepassten, kragenartigen Klebe- bzw. Schweissrand (14) aufweist, der im fertig montierten Zustand mit der Klebe- bzw. Schweisswand (13) dichtend verbunden ist.

8. Larynxmaske (1) nach Anspruch 5, **dadurch gekennzeichnet, dass** der Cuff (12') hohlraumfrei aus vollem Material besteht.

9. Larynxmaske (1) nach Anspruch 8, **dadurch gekennzeichnet, dass** im Cuff (12'') an der dorsalen Seite mindestens entlang einer der lateralen Ränder des Cuffs mindestens eine in Längsrichtung verlaufende, eine die Komprimierbarkeit in medial-lateraler Richtung erhöhende Federrinne (15) eingeformt ist.

10. Larynxmaske (1) nach Anspruch 9, **dadurch gekennzeichnet, dass** entlang beider lateralen Wände (16) des Cuffs (12'') und mindestens annähernd parallel zu denselben mindestens je eine Federrinne (15) eingeformt ist.

11. Larynxmaske (1) nach Anspruch 1, **dadurch gekennzeichnet, dass** das Führungslumen (6) in einem Übergangsbereich (18) beim proximalen Ende (7) des Tubus (2) zum Larynxmaskenkopf (3) geschlossen ist, während in denselben Bereich das Respirationslumen (4) in den Respirationsraum (10) übergeht, während in demselben Übergangsbereich (18) an Stelle an der das Oesophageallumen (5) in den Oesophagealdurchgang (11) übergeht hier eine Richtungsänderungsstelle (19) besteht.

12. Spritzgussformwerkzeug zur Fertigung einer Larynxmaske (1) mit supraglottischem Tubus (2), gemäss mindestens einem der Ansprüche 1 - 11, **dadurch gekennzeichnet, dass** zur Bildung des Tubus (2) drei parallele Kerne vorhanden sind, wobei der Kern zur Bildung des Führungslumens (6) und einer der benachbarten Kerne vom proximalen Ende an in distaler Richtung über einen Stabilisierungssteg miteinander verbunden sind.

13. Spritzgussformwerkzeug nach Anspruch 12, **dadurch gekennzeichnet, dass** zwei den Respirationsraum und den darüber verlaufenden Oesophagealdurchgang formende Kerne Teile einer ersten Spritzgusswerkzeughälfte und die drei die Lumen des Tubus bildende Kerne, Teil einer zweiten Spritzgusshälfte sind.

14. Spritzgussformwerkzeug nach Anspruch 12, **dadurch gekennzeichnet, dass** der das Oesophageallumen bildende Kern und der den Oesophagealdurchgang bildende Kern im Schliessbereich der beiden Spritzgussformwerkzeughälften formschlüssig in Schliessbewegungsrichtung ineinandergreifen.

15. Spritzgussformwerkzeug nach Anspruch 13, **dadurch gekennzeichnet, dass** der den Respirationsraum bildende Kern und der das Respirationslumen bildende Kern im Schliessbereich der beiden Spritzgussformwerkzeughälften formschlüssig in Schliessbewegungsrichtung ineinandergreifen.

16. Spritzgussformwerkzeug nach Anspruch 12, **dadurch gekennzeichnet, dass** die die Lumen bildenden Kerne als bewegliche Schieber oder Züge gebildet sind.

## Claims

1. A laryngeal mask (1) having a supraglottic tube (2) for insertion of a laryngeal mask head (3) over the larynx, wherein the supraglottic tube has three lumens, namely a respiration lumen (4), which serves to supply respiration air and for instrumentation, an esophageal lumen (5), which serves to provide esophageal access, and a guide lumen (6) that runs medially in between the two former lumens and is closed at the proximal end,
**characterized in that**
the supraglottic tube (2) and the laryngeal mask head (3) are manufactured by injection molding in one piece, wherein a slot (8), which connects the two lumens runs over at least a portion of the length of the supraglottic tube (2) from the distal end (9) the slot runs between the lateral lumen (4,5) and the medial guide lumen (6).

2. The laryngeal mask (1) according to Claim 1,
**characterized in that**
the connecting slot (8) connects the respiration lumen (4) with the guide lumen (6).

3. The laryngeal mask (1) according to Claim 1,
**characterized in that**
the connecting slot (8) connects the esophageal lumen (5) to the guide lumen (6).

4. The laryngeal mask (1) according to Claim 1,
**characterized in that**
the length of the slot (8) extends at least over one-fourth, preferably at least one-third of the total length of the tube (2) from the proximal end (7) to the distal end (9).

5. The laryngeal mask (1) according to Claim 1,
**characterized in that**
the one-piece laryngeal mask (1) has in the laryngeal mask head (3) a central respiration space (10), in which the respiration lumen (4) of the tube (2) ends, and above the respiration space (10) there is an esophageal passage (11), in which the esophageal lumen (5) opens, and the respiration space (10) is surrounded by a cuff (12).

6. The laryngeal mask (1) according to Claim 5,
**characterized in that**
the cuff (12) is an inflatable cuff.

7. The laryngeal mask (1) according to Claim 5,
**characterized in that**
the respiration space (10) is bordered by a peripheral adhesive wall and/or welded wall (13), and the open injected cuff (12) has a peripheral collar type adhesive edge and/or welded edge (14), which is adapted to the shape and size of the adhesive wall and/or welded wall (13) and in the completely assembled state is connected to the adhesive wall and/or welded wall (13) with a seal.

8. The laryngeal mask (1) according to Claim 5,
**characterized in that**
the cuff (12') consists of solid material without any cavities.

9. The laryngeal mask (1) according to Claim 8,
**characterized in that**
at least one spring channel (15), which increases the compressibility in the medial-lateral direction is molded on the dorsal side of the cuff (12'') running at least in the longitudinal direction along at least one of the lateral edges of the cuff.

10. The laryngeal mask (1) according to Claim 9,
**characterized in that**
at least one spring channel (15) is molded along each of the two lateral walls (16) of the cuff (12''), at least approximately parallel to same.

11. The laryngeal mask (1) according to Claim 1,
**characterized in that**
the guide lumen (6) is closed with respect to the laryngeal mask head (3) in a transitional area (18) on the proximal end (7) of the tube (2), while the respiration lumen (4) develops into the respiration space (10) in the same region, while a location of a change in direction (19) exists in the same transitional area (18) at the location where the esophageal lumen (5) develops into the esophageal passage (11).

12. An injection mold for production of a laryngeal mask (1) having a supraglottic tube (2) according to at least one of Claims 1-11,
**characterized in that**
to form the tube (2) there are three parallel cores, wherein the core to form the guide lumen (6) and one of the neighboring cores are connected to one another by means of a stabilizing web from the proximal end in the distal direction.

13. The injection mold according to Claim 12,
**characterized in that**
two cores forming the respiration space and the esophageal passage running above it are parts of a first injection mold half, and the three cores forming the lumens of the tube are part of a second injection mold half.

14. The injection mold according to Claim 12,
**characterized in that**
the core forming the esophageal lumen and the core forming the esophageal passage engage in one another in a form-fitting manner in the direction of the closing movement in the closing region of the two injection mold halves.

15. The injection mold according to Claim 13,
**characterized in that**
the core forming the respiration space and the core forming the respiration lumen engage in one another in a form-fitting manner in the direction of the closing movement in the closing region of the two injection mold halves.

16. The injection mold according to Claim 12,
**characterized in that**
the cores forming the lumens are formed as movable slides or pulls.

## Revendications

1. Masque laryngé (1) comprenant un tube supraglottique (2) destiné à l'introduction d'une tête de masque laryngé (3) par le larynx, sachant que le tube supraglottique présente trois lumières, à savoir une lumière respiratoire (4) qui sert à l'arrivée d'air respiratoire et à l'instrumentation, une lumière oesophagienne (5) qui sert à accéder à l'oesophage et une lumière de guidage (6) médiane entre ceux-ci fermée au niveau proximal, **caractérisé en ce que** le tube supraglottique (2) et la tête de masque laryngé (3) sont fabriqués d'une seule pièce par moulage par injection, sachant qu'entre une lumière latérale (4, 5) et la lumière de guidage médiane (6) passe une fente (8) reliant les deux lumières sur au moins une partie de la longueur du tube supraglottique (2) depuis l'extrémité distale (9).

2. Masque laryngé (1) selon la revendication 1, **caractérisé en ce que** la fente de liaison (8) relie la lumière respiratoire (4) à la lumière de guidage (6).

3. Masque laryngé (1) selon la revendication 1, **caractérisé en ce que** la fente de liaison (8) relie la lumière oesophagienne (5) à la lumière de guidage (6).

4. Masque laryngé (1) selon la revendication 1, **caractérisé en ce que** la longueur de la fente (8) s'étend sur au moins un quart, de préférence au moins un tiers de la longueur totale du tube (2), de l'extrémité proximale (7) à l'extrémité distale (9).

5. Masque laryngé (1) selon la revendication 1, **caractérisé en ce que** le masque laryngé (1) d'une seule pièce présente un espace respiratoire central (10) dans la tête de masque laryngé (3), dans lequel la lumière respiratoire (4) du tube (2) débouche et un passage oesophagien (11) passe au-dessus de l'espace respiratoire (10), dans lequel la lumière oesophagienne (5) débouche, et que l'espace respiratoire (10) est entouré par un ballonnet (12).

6. Masque laryngé (1) selon la revendication 5, **caractérisé en ce que** le ballonnet (12) est un ballonnet gonflable.

7. Masque laryngé (1) selon la revendication 5, **caractérisé en ce que** l'espace respiratoire (10) est délimité par une paroi collée, respectivement soudée (13) périphérique et que le ballonnet (12) injecté ouvert présente un bord collé, respectivement soudé (14) de type collet, adapté à la forme et à la taille de la paroi collée, respectivement soudée (13), lequel est relié de façon étanche à la paroi collée, respectivement soudée (13) dans l'état monté et prêt.

8. Masque laryngé (1) selon la revendication 5, **caractérisé en ce que** le ballonnet (12') est composé d'un matériau plein sans vide.

9. Masque laryngé (1) selon la revendication 8, **caractérisé en ce qu'**au moins une goulotte élastique (15) augmentant la capacité de compression dans le sens médial-latéral, passant au moins dans le sens longitudinal, au moins le long d'un des bords latéraux du ballonnet, est formée dans le ballonnet (12''), sur le côté dorsal.

10. Masque laryngé (1) selon la revendication 9, **caractérisé en ce qu'**au moins une goulotte élastique (15) est formée le long de chacune des deux parois latérales (16) du ballonnet (12'') et au moins presque parallèlement à celles-ci.

11. Masque laryngé (1) selon la revendication 1, **caractérisé en ce que** la lumière de guidage (6) est fermée dans une zone de transition (18) à l'extrémité proximale (7) du tube (2) vers la tête de masque laryngé (3), tandis que dans la même zone, la lumière respiratoire (4) passe dans l'espace respiratoire (10), tandis que dans la même zone de transition (18) à l'endroit où la lumière oesophagienne (5) passe dans le passage oesophagien (11), il y a un point de changement de sens (19).

12. Outil de moulage par injection destiné à la fabrication d'un masque laryngé (1) comprenant un tube supraglottique (2), selon au moins l'une des revendications 1 - 11, **caractérisé en ce que** pour former le tube (2), trois noyaux parallèles sont présents, sachant que le noyau pour former la lumière de guidage (6) et un des noyaux voisins sont reliés entre eux par le biais d'une tige de stabilisation, de l'extrémité proximale dans le sens distal.

13. Outil de moulage par injection selon la revendication 12, **caractérisé en ce que** deux noyaux formant l'espace respiratoire et le passage oesophagien passant dessus, font partie d'une première moitié d'outil de moulage par injection et les trois noyaux formant les lumières du tube font partie d'une seconde moitié d'outil de moulage par injection.

14. Outil de moulage par injection selon la revendication 12, **caractérisé en ce que** le noyau formant la lumière oesophagienne et le noyau formant le passage oesophagien s'engagent l'un dans l'autre au niveau de la fermeture des deux moitiés d'outil de moulage par injection par complémentarité de forme, dans le sens du mouvement de fermeture.

15. Outil de moulage par injection selon la revendication 13, **caractérisé en ce que** le noyau formant l'espace respiratoire et le noyau formant la lumière respiratoire s'engagent l'un dans l'autre au niveau de la fermeture des deux moitiés d'outil de moulage par injection par complémentarité de forme dans le sens du mouvement de fermeture.

16. Outil de moulage par injection selon la revendication 12, **caractérisé en ce que** les noyaux formant les lumières sont formés en tant que poussoirs ou tiroirs mobiles.
